# FASCICULE DE BREVET EUROPEEN

(11) **EP 1 265 698 B1**
(45) Date de publication et mention de la délivrance du brevet: **16.06.2004**
(21) Numéro de dépôt: 01911828.0
(22) Date de dépôt: 02.03.2001
(51) Int. Cl.: B01J 13/00, B01F 17/00, A61K 9/51, B01J 13/06

(54) **NANOCAPSULES LIPIDIQUES, PROCEDE DE PREPARATION ET UTILISATION COMME MEDICAMENT**
LIPIDNANOKAPSELN, SEINE HERSTELLUNGSVERFAHREN SOWIE VERWENDUNG ALS MEDIZIN
LIPID NANOCAPSULES, PREPARATION METHOD AND USE AS MEDICINE

(30) Priorité: 02.03.2000 FR 0002688
(43) Date de publication de la demande: 18.12.2002
(73) Titulaire: MAINELAB, 49100 Angers (FR); UNIVERSITE D'ANGERS, 49000 Angers (FR)
(72) Inventeur: HEURTAULT, Béatrice, F-49100 Angers (FR); SAULNIER, Patrick, F-49130 Les-Ponts-de-Cé (FR); BENOIT, Jean-Pierre, F-49240 Avrille (FR); PROUST, Jacques-Emile, F-49170 Saint-Léger-des-Bois (FR); PECH, Brigitte, F-49100 Angers (FR); RICHARD, Joel, F-49160 Longue (FR)
(74) Mandataire: Warcoin, Jacques
(86) Numéro de dépôt international: PCT/FR2001/000621
(87) Numéro de publication internationale: WO 2001/064328

(56) Documents cités:
- EP-A- 0 447 318
- EP-A- 0 529 711
- EP-A- 0 621 073
- EP-A- 0 717 989
- FR-A- 2 692 167
- US-A- 5 049 322
- US-A- 5 174 930

## Description

La présente invention a pour objet des nanocapsules lipidiques, un procédé pour leur préparation et leur utilisation pour la fabrication d'un médicament, destiné notamment à être administré par voie injectable, orale ou nasale.

Ces dernières années, de nombreux groupes ont développé la formulation de nanoparticules solides lipidiques ou nanosphères lipidiques (Müller, R.H. et Mehnert, European Journal of Pharmaceutics and Biopharmaceutics, 41 (1 ) : 62-69, 1995 ; W., Gasco, M.R., Pharmaceutical Technology Europe : 52-57, décembre 1997; EP 605 497). Il s'agit d'une alternative à l'utilisation des liposomes ou des particules polymères. Ces particules lipidiques présentent l'avantage d'être formulées en l'absence de solvant. Elles ont permis d'encapsuler à la fois des produits lipophiles et hydrophiles sous forme de paires d'ions par exemple (Cavalli, R. et al, S.T.P: Pharma Sciences, 2(6): 514-518,1992; et Cavalli; R. et al, International Journal of Pharmaceutics, 117: 243-246, 1995). Ces particules peuvent être stables sur plusieurs années à l'abri de la lumière, à 8°C (Freitas, C. et Müller, R.H., Journal of Microencapsulation, 1 (16): 59-71, 1999).

Deux techniques sont couramment utilisées pour préparer des nanoparticules lipidiques :
- l'homogénéisation d'une émulsion chaude (Schwarz, C. et al, Journal of Controlled Release, 30 : 83-96, 1994 ; Müller, R.H. et al, European Journal of Pharmaceutics and Biopharmaceutics, 41(1) : 62-69, 1995) ou froide (Zur Mühlen, A. and Mehnert W., Pharmazie, 53 : 552-555, 1998 ; EP 605 497), ou
- la trempe d'une microémulsion en présence de co-tensioactifs tels que le butanol. La taille des nanoparticules obtenues est en général supérieure à 100 nm (Cavalli, R. et al, European Journal of Pharmaceutics and Biopharmaceutics, 43(2): 110-115, 1996; Morel, S. et al, international Journal of Pharmaceutics, 132 : 259-261, 1996).

Cavalli et al. (International Journal of Pharmaceutics, 2(6) : 514-518, 1992 ; et Pharmazie, 53 : 392-396, 1998) décrivent l'utilisation d'un sel biliaire, le taurodéoxycholate, non toxique par voie injectable pour la formation de nanosphères de taille supérieure ou égale à 55 nm.

La présente invention concerne des nanocapsules et non des nanosphères. On entend par nanocapsules des particules constituées d'un coeur liquide ou semi-liquide à température ambiante, enrobé d'un film solide à température ambiante, par opposition à des nanosphères qui sont des particules matricielles, i.e. dont la totalité de la masse est solide. Lorsque les nanosphères contiennent un principe pharmaceutiquement actif, celui-ci est finement dispersé dans la matrice solide.

Dans le cadre de la présente invention, on entend par température ambiante, une température comprise entre 15 et 25°C.

La présente invention a pour objet des nanocapsules de taille moyenne inférieure à 150 nm, de préférence inférieure à 100 nm, de préférence encore inférieure à 50 nm. Les nanocapsules sont chacunes constituées d'un coeur essentiellement lipidique liquide ou semi-liquide à température ambiante, enrobé d'un film essentiellement lipidique solide à température ambiante.

Compte-tenu de leur taille, les nanocapsules de l'invention sont des particules lipidiques colloïdales.

L'indice de polydispersité des nanocapsules de l'invention est avantageusement compris entre 5 et 15 %.

L'épaisseur du film solide est avantageusement comprise entre 2 à 10 nm. Elle est égale environ au dixième du diamètre des particules.

Le coeur des nanocapsules est essentiellement constitué d'un corps gras liquide ou semi-liquide à température ambiante, par exemple un triglycéride ou un ester d'acide gras, représentant 20 à 60 %, préférentiellement 25 à 50 % en poids des nanocapsules.

Le film solide enrobant les nanocapsules est, de préférence, essentiellement constitué d'un tensio-actif lipophile, par exemple une lécithine dont la proportion en phosphatidylcholine est comprise entre 40 et 80 %. Le film solide peut également contenir un tensio-actif hydrophile, par exemple le Solutol® HS 15.

Le tensio-actif hydrophile contenu dans le film solide enrobant les nanocapsules représente de préférence entre 2 à 10 % en poids des nanocapsules, de préférence 8 % environ.

Le triglycéride constituant le coeur des nanocapsules est notamment choisi parmi les triglycérides en C₈ à C₁₂, par exemple des triglycérides des acides capriques et capryliques et leurs mélanges.

L'ester d'acide gras est choisi parmi les esters d'acide gras en C₈ à C₁₈, par exemple le palmitate d'éthyle, l'oléate d'éthyle, le myristate d'éthyle, le myristate d'isopropyle, le myristate d'octyldodécyle, et leurs mélanges. L'ester d'acide gras est de préférence en C₈ à C₁₂.

Les nanocapsules de l'invention sont particulièrement adaptées à la formulation de principes actifs pharmaceutiques. Dans ce cas, le tensio-actif lipophile peut être avantageusement solide à 20°C et liquide à 37°C environ.

La quantité de tensio-actif lipophile contenu dans le film solide enrobant les nanocapsules est fixée de telle sorte que le rapport massique corps gras liquide / composé tensio-actif solide est choisi entre 1 et 15, de préférence entre 1,5 et 13, plus préférentiellement entre 3 et 8.

La présente invention a également pour objet un procédé de préparation des nanocapsules décrites précédemment.

Le procédé de l'invention est basé sur l'inversion de phase d'une émulsion huile/eau provoquée par plusieurs cycles de montée et de descente en température.

Le procédé de l'invention consiste à
a)
   - préparer une émulsion huile/eau contenant une phase grasse huileuse, un tensio-actif hydrophile non ionique, un tensio-actif lipophile solide à 20°C, et éventuellement un principe pharmaceutiquement actif, soluble ou dispersible en phase grasse huileuse, ou un principe pharmaceutiquement actif soluble ou dispersible en phase aqueuse.
   - provoquer l'inversion de phase de ladite émulsion huile/eau par augmentation de la température jusqu'à une température T₂ supérieure à la température d'inversion de phase (TIP) pour obtenir une émulsion eau/huile, suivie d'une diminution de la température jusqu'à une température T₁, T₁ < TIP < T₂.
   - effectuer au moins un ou plusieurs cycles de température autour de la zone d'inversion de phase entre T₁ et T₂, jusqu'à observer une suspension translucide,
b) effectuer la trempe de l'émulsion huile/eau à une température voisine de T₁, de préférence supérieure à T₁, pour obtenir des nanocapsules stables.

Les nanocapsules obtenues selon le procédé de l'invention sont avantageusement dépourvues d'agents co-tensio-actifs, comme les alcools en C₁ - C₄.

Le nombre de cycles appliqués à l'émulsion dépend de la quantité d'énergie nécessaire pour former les nanocapsules.

L'inversion de phase peut être visualisée par l'annulation de la conductivité de la formulation lorsque l'émulsion eau/huile se forme.

Le procédé de l'invention comprend deux étapes.

La première étape consiste à peser l'ensemble des constituants, à les chauffer au delà d'une température T₂ sous agitation douce (par exemple magnétique) puis éventuellement à les refroidir à une température T₁ (T₁<T₂). Après un certain nombre de cycles de température, on obtient une émulsion eau/huile.

L'inversion de phase entre l'émulsion huile/eau et l'émulsion eau/huile se traduit par une diminution de la conductivité quand la température augmente jusqu'à ce qu'elle s'annule. La température moyenne de la zone d'inversion de phase correspond à la température d'inversion de phase (TIP). L'organisation du système sous forme de nanocapsules se traduit visuellement par un changement d'aspect du système initial qui passe de blanc-opaque à blanc-translucide. Ce changement se produit à une température inférieure à la TIP. Cette température est située généralement entre 6 à 15°C en dessous de la TIP.

T₁ est une température à laquelle la conductivité est au moins égale à 90 - 95 % de la conductivité mesurée à 20°C.

T₂ est la température à laquelle la conductivité s'annule.

La deuxième étape consiste en un refroidissement brusque (ou trempe) de l'émulsion huile/eau à une température voisine de T₁, de préférence supérieure à T₁, sous agitation magnétique, en la diluant entre 3 et 10 fois à l'aide d'eau désionisée à 2°C +/- 1°C jetée dans l'émulsion fine. Les particules obtenues sont maintenues sous agitation pendant 5 min.

Dans un mode de réalisation préférée, la phase grasse est un triglycéride d'acide gras, le tensio-actif lipophile solide est une lécithine et le tensio-actif hydrophile est le Solutol® HS15. Dans ces conditions, T₁ = 60°C, T₂= 85°C et le nombre de cycles est égal à 3.

Le rapport corps liquide / composé tensio-actif solide est choisi entre 1 et 15, de préférence entre 1,5 et 13, plus préférentiellement entre 3 et 8.

L'émulsion huile/eau contient avantageusement 1 à 3 % de tensio-actif lipophile, 5 à 15 % de tensio-actif hydrophile, 5 à 15 % de corps gras huileux, 64 à 89 % d'eau (les pourcentages sont exprimés en poids).

Plus l'indice HLB du corps gras liquide est élevé, plus la température d'inversion de phase est élevée. En revanche, la valeur de l'indice HLB du corps gras ne semble pas avoir d'influence sur la taille des nanocapsules.

Ainsi, lorsque la taille des groupements terminaux des triglycérides augmente, leur indice HLB diminue et la température d'inversion de phase diminue.

L'indice HLB ou balance hydrophile-lipophile est tel que défini par C. Larpent dans le Traité K.342 des Editions TECHNIQUES DE L'INGENIEUR.

La taille des particules diminue quand la proportion en agent tensio-actif hydrophile augmente et quand la proportion en agents tensio-actifs (hydrophile et lipophile) augmente. En effet, l'agent tensio-actif entraîne une diminution de la tension interfaciale et donc une stabilisation du système ce qui favorise l'obtention de petites particules.

Par ailleurs, la taille des particules augmente quand la proportion d'huile augmente.

Selon un mode de réalisation préférée, la phase grasse est le Labrafac® WL 1349, le tensio-actif lipophile est le Lipoïd® S 75-3 et le tensio-actif hydrophile non ionique est le Solutol® HS 15. Ces composés présentent les caractéristiques suivantes :
- Le Labrafac® lipophile WL1349 (Gattefossé, Saint-Priest, France). Il s'agit d'une huile composée de triglycérides à chaîne moyenne des acides capryliques et capriques (C₈ et C₁₀). Sa densité est de 0,930 à 0,960 à 20°C. Son indice HLB est de l'ordre de 1.
- Le Lipoïd® S 75-3 (Lipoid GmbH, Ludwigshafen, Allemagne). Le Lipoïd® S 75-3 correspond à de la lécithine de soja. Cette dernière contient environ 69 % de phosphatidylcholine et 9 % de phosphatidyl éthanolamine. Ce sont donc des composés tensio-actifs. Ce constituant est le seul constituant solide à 37°C et à température ambiante dans la formulation, Il est couramment utilisé pour la formulation de particules injectables.
- Le Solutol® HS 15 (Basf, Ludwigshafen, Allemagne). Il s'agit d'un 2-hydroxystéarate de polyéthylèneglycol-660. Il joue donc le rôle d'agent tensio-actif hydrophile non ionique dans la formulation, Il est utilisable par voie injectable (chez la souris en IV DL50 > 3,16 g/kg, chez le rat 1,0 < DL 50 < 1,47 g /kg).

La phase aqueuse de l'émulsion huile/eau peut également contenir 1 à 4 % d'un sel comme le chlorure de sodium. La modification de la concentration en sel entraîne un déplacement de la zone d'inversion de phase. Plus la concentration en sel augmente et plus la température d'inversion de phase est basse. Ce phénomène sera intéressant pour l'encapsulation de principes actifs thermosensibles hydrophobes. Leur incorporation pourra se faire à une température plus faible.

Les nanocapsules de l'invention peuvent avantageusement contenir un principe actif et entrer dans la composition d'un médicament destiné à être administré par voie injectable, notamment intra-veineuse, par voie orale ou par voie nasale.

Lorsque le principe actif est peu soluble dans la phase huileuse, on ajoute un co-solvant, par exemple la N,N-diméthylacétamide.

Les nanocapsules de l'invention conviennent plus particulièrement pour l'administration des principes actifs suivants :
- les anti-infectieux parmi lesquels les antimycosiques, les antibiotiques,
- les anticancéreux,
- les principes actifs destinés au Système Nerveux Central qui doivent passer la barrière hémato-encéphalique, tels que les antiparkinsoniens et plus généralement les principes actifs pour traiter les maladies neurodégénératives.

Le principe pharmaceutiquement actif peut être tout d'abord soluble ou dispersible en phase grasse huileuse, et dans ce cas, il sera incorporé dans le coeur de la nanocapsule. Pour ce faire, il est incorporé au stade de la première étape de préparation de l'émulsion huile/eau qui contient en outre, la phase grasse huileuse, un tensio-actif hydrophile non-ionique et un tensio-actif lipophile solide à 20°C.

Le principe pharmaceutiquement actif peut également être de nature hydrosoluble ou dispersible en phase aqueuse, et dans pareil cas, il ne sera fixé à la surface des nanocapsules qu'à l'issue de la phase ultime de préparation des nanocapsules stables. Un tel principe actif hydrosoluble peut être de toute nature, y compris les protéines, les peptides, les oligonucléotides et les plasmides ADN. Un tel principe actif est fixé à la surface des nanocapsules par introduction dudit principe actif dans la solution au sein de laquelle se trouvent dispersées les nanocapsules stables obtenues à l'issue du procédé selon l'invention. La présence d'un agent tensio-actif hydrophile non-ionique favorise les liaisons d'interaction entre le principe actif hydrosoluble et la surface libre des nanocapsules.

Le principe actif hydrosoluble pourra également être introduit dans la phase aqueuse lors de la première étape de préparation initiale huile/eau.

La présente invention est illustrée par les exemples suivants en référence aux figures 1 à 4.

La figure 1 est une photographie des nanocapsules de l'invention obtenues dans l'exemple 1. L'échelle est de 1 cm pour 50 nm.

La figure 2 représente l'évolution de la taille moyenne des particules en fonction de la proportion en agent tensio-actif hydrophile (Solutol® ).

La figure 3 représente l'évolution de la conductivité en fonction de la température pour différentes concentrations en sel. Dans la courbe 1, la concentration en sel est de 2,0 % en poids. Dans la courbe 2, la concentration est de 3,4 % en poids.

La figure 4 représente l'évolution de la conductivité d'une émulsion huile/eau (H/E) décrite dans l'exemple 1, en fonction de la température après trois cycles de montée et descente en température entre 60 et 85°C.

### Exemple 1 : Nanocapsules non chargées en principe actif

### A) Préparation des nanocapsules

On réalise 5 g d'une émulsion contenant 75 mg de Lipoïd® S75-3, 504 mg de Labrafac® WL 1349 lipophile, 504 mg de Solutol® HS 15, 3,829 g d'eau et 88 mg de chlorure de sodium.

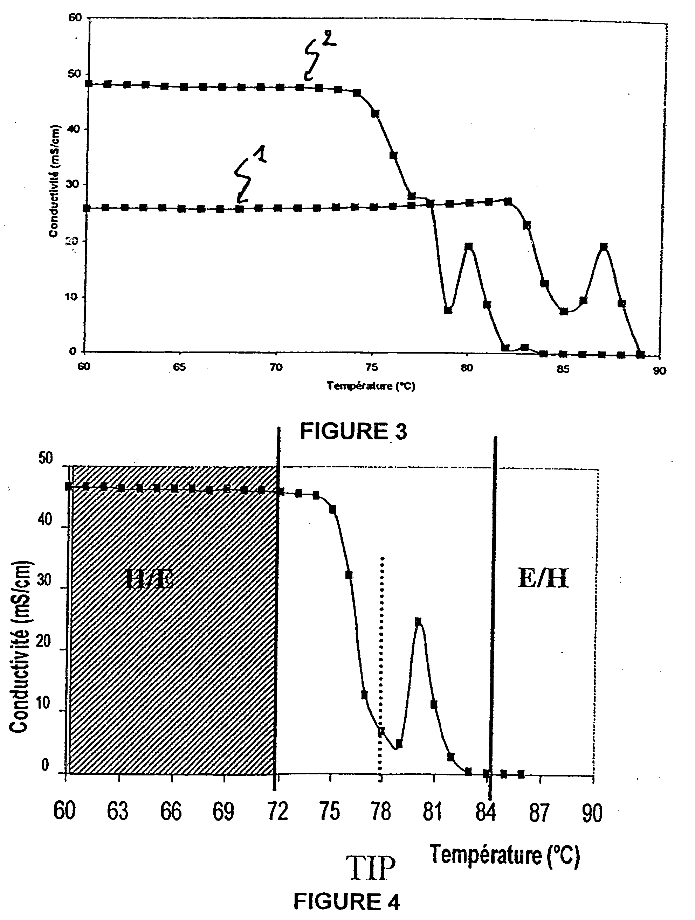

L'ensemble est réuni dans un même bêcher et placé sous agitation magnétique. Un chauffage est appliqué jusqu'à atteindre une température de 85°C. Toujours sous agitation magnétique, on laisse refroidir le système jusqu'à une température de 60°C. Ce cycle (entre 85°C et 60°C) est réalisé jusqu'à ce que l'on observe l'annulation de la conductivité en fonction de la température (figure 4). L'inversion de phase se produit au bout de trois cycles. Au dernier refroidissement, on effectue une trempe en jetant 12,5 ml d'eau distillée à 2°C +/- 1°C sur le mélange à 70°C. Le système est alors maintenu sous agitation magnétique pendant 5 min.

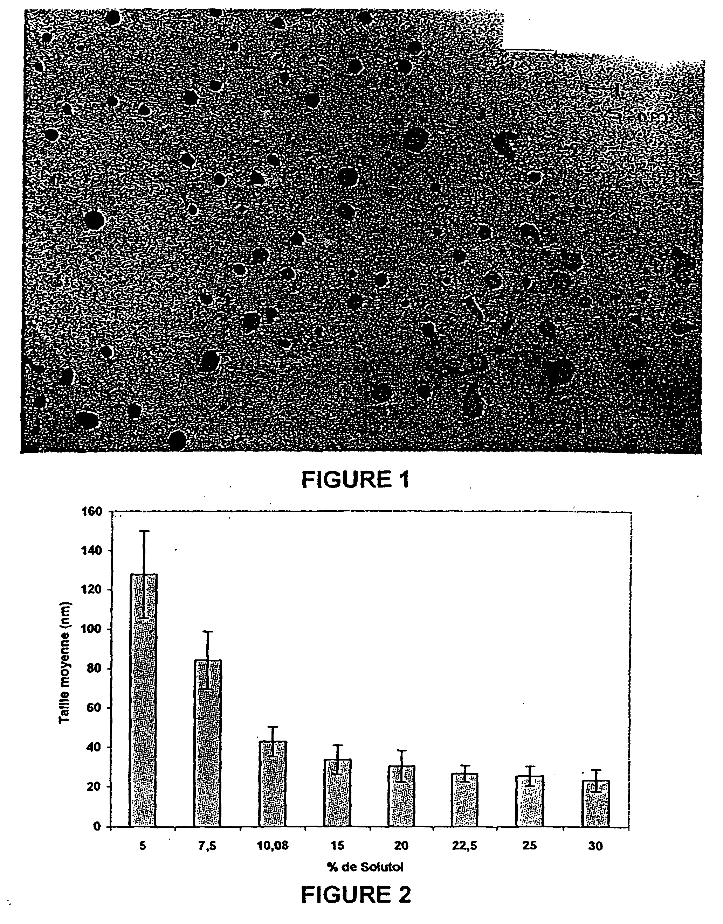

Les particules obtenues dans les conditions précédemment décrites, après trois cycles de température, ont une taille moyenne de 43 +/- 7 nm. Leur polydispersité de taille est de 0,071. La microscopie électronique à transmission en utilisant l'acide phosphotungstique nous a permis de mettre en évidence des particules de taille moyenne de l'ordre de 50 nm (voir figure 1). Par ailleurs, une observation faite en microscopie de force atomique en mode contact (appareillage Park Scientic Instruments, Genève, Suisse) montre que les nanocapsules sont effectivement solides à la température de 25°C.

### B) Modification des proportions d'agent tensio-actif hydrophile

Le tableau I ci-dessous présente différentes formulations de nanocapsules préparées avec des concentrations variables en tensio-actif hydrophile.

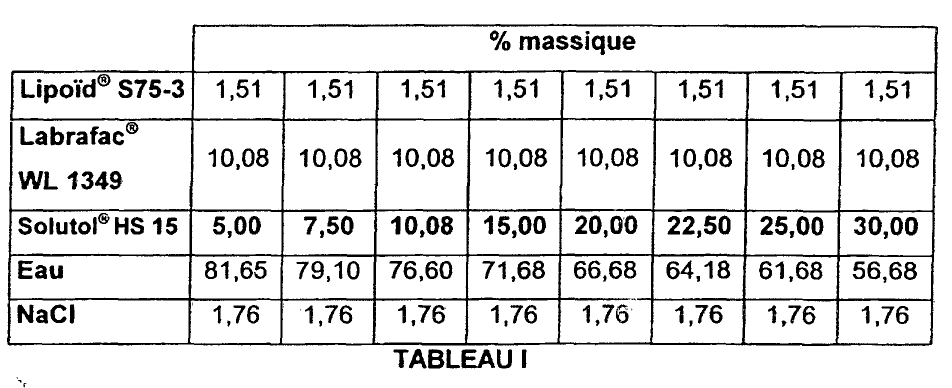

La diminution de la concentration en Solutol® HS 15 entraîne une augmentation de la taille moyenne des particules (figure 2). On observe alors des tailles moyennes qui vont de 23 à 128 nm pour des proportions de Solutol® allant de 30 à 5 % de la formulation totale respectivement. La taille dépend donc de la concentration en agent tensioactif hydrophile.

### C) Modifications des proportions d'agents tensio-actifs Lipoïd® et Solutol®

Le tableau II ci-dessous présente des formulations de nanocapsules préparées avec différentes concentrations d'agents tensio-actifs.

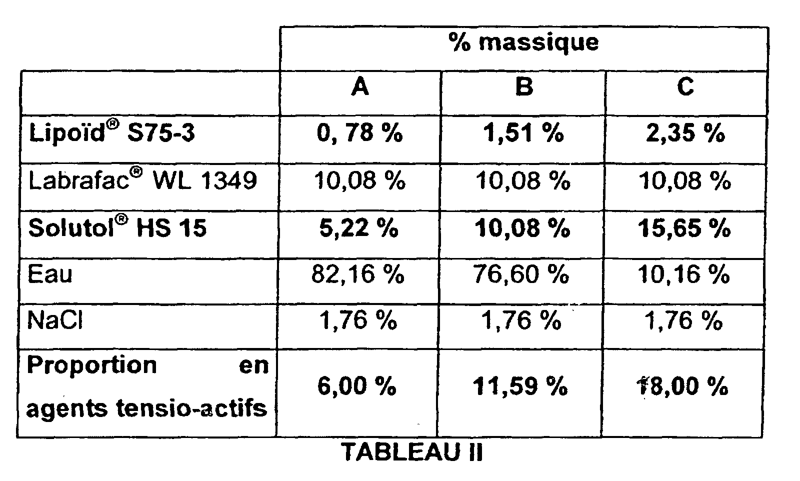

L'augmentation de la proportion d'agents tensioactifs dans la formulation entraîne une diminution de la taille moyenne. En effet, la formulation A permet d'obtenir des particules de taille moyenne 85 +/- 7 nm (P = 0,124). Pour les formulations B et C les tailles moyennes deviennent 43 +/- 7 nm (P = 0,071), et 29 +/- 8 nm (P = 0,148) respectivement.

### D) Modification de la concentration en NaCl

Le tableau III ci-dessous présente deux formulations de nanocapsules préparées avec deux concentrations en sel NaCI différentes.

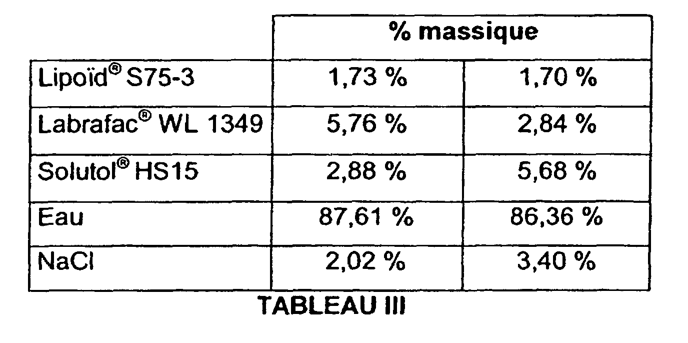

La modification de la concentration en sel entraîne un déplacement de la zone d'inversion de phase. Plus la concentration en sel augmente et plus la température d'inversion de phase est basse (figure 3). Ce phénomène sera intéressant pour l'encapsulation de principes actifs thermosensibles hydrophobes. Leur incorporation pourra se faire à une température plus faible.

Ces formulations permettent d'obtenir des particules de taille similaire aux tailles précédentes malgré les concentrations en sel différentes.

### Exemple 2 : Encapsulation d'un principe actif lipophile, le Soudan III

La formulation correspond à celle de l'exemple 1 : on réalise 5 g de l'émulsion initiale en pesant 75 mg de Lipoïd® S75-3, 504 mg de Labrafac® lipophile et 504 mg de Solutol® , 3,829 g d'eau et 88 mg de chlorure de sodium. On ajoute 200 mg de Soudan III solubilisé dans de l'huile de vaseline. L'ensemble est pesé dans un même bêcher et placé sous agitation magnétique. Un chauffage est appliqué jusqu'à atteindre une température de 85°C. Toujours sous agitation magnétique, on laisse refroidir le système jusqu'à une température de 60°C. Ce cycle (entre 85°C et 60°C) est réalisé trois fois. Au dernier refroidissement on effectue une trempe à 70°C en jetant 12,5 ml d'eau distillée à 2°C +/- 1°C. Le système est alors maintenu sous agitation magnétique pendant 5 min.

L'encapsulation de Soudan III nous a permis d'obtenir des particules de taille similaire aux particules de l'exemple 1, pour les mêmes proportions de tensioactifs et de phase grasse, soit 45 +/- 12 nm (P = 0,138). A l'oeil nu, l'échantillon apparaît rose homogène.

### Exemple 3 : Encapsulation de la progestérone.

La formulation correspond à celle de l'exemple 1 : on réalise 5 g de l'émulsion initiale en pesant 75 mg de Lipoïd® S75-3. 504 mg de Labrafac® lipophile et 504 mg de Solutol® , 3,829 g d'eau et 88 mg de chlorure de sodium. On ajoute 10 mg de progestérone. L'ensemble est pesé dans un même bêcher et placé sous agitation magnétique. Un chauffage est appliqué jusqu'à atteindre une température de 85°C. Toujours sous agitation magnétique, on laisse refroidir le système jusqu'à une température de 60°C. Ce cycle (entre 85°C et 60°C) est réalisé trois fois. Au dernier refroidissement, on effectue une trempe à 70°C en jetant 12,5 ml d'eau distillée à 2°C +/- 1°C. Le système est alors maintenu sous agitation magnétique pendant 5 min.

L'encapsulation de progestérone permet d'obtenir des particules de tailles similaires aux particules de l'exemple 1, à savoir 45 +/- 12 nm (P = 0,112). La progestérone n'est pas retrouvée dans la phase aqueuse à une concentration supérieure à sa solubilité. En effet, une centrifugation à 200000 tr/min pendant 30 minutes permet d'obtenir un léger précipité dont la composition a été étudiée par DSC. Ce précipité ne contient pas de progestérone. La progestérone étant quasiment insoluble dans l'eau, cela indique une incorporation du principe actif dans les nanocapsules.

### Exemple 4 : Encapsulation d'une suspension de busulfan

### A) Suspension de busulfan (à une concentration de 0,25 mg/ml)

La première étape de l'encapsulation de busulfan consiste à solubiliser celui-ci dans de la N,N-diméthyl-acétamide. On réalise donc une solution à 24 mg de busulfan par mL de N,N diméthyl acétamide. On prélève 175 mg de cette solution que l'on ajoute aux 504 mg de Labrafac® . On pèse également 75 mg de Lipoïd® S75-3, 504 mg de Solutol® , 3,829 g d'eau et 88 mg de chlorure de sodium. L'émulsion initiale est donc à une concentration de 0,88 mg/g d'émulsion. L'ensemble est réuni dans un même bêcher et placé sous agitation magnétique. Un chauffage est appliqué jusqu'à atteindre une température de 85°C. Toujours sous agitation magnétique, on laisse refroidir le système jusqu'à une température de 60°C. Ce cycle (entre 85°C et 60°C) est réalisé trois fois. Au demier refroidissement, on effectue une trempe à 70°C en jetant 12,5 ml d'eau distillée à 2°C +/- 1°C. Le système est alors maintenu sous agitation magnétique pendant 5 min. La concentration finale, c'est-à-dire après trempe donc dilution, est de 0,25 mg/ ml.

La taille des particules obtenues est légèrement supérieure à celle de l'exemple 1 en raison de la proportion plus importante de phase grasse (63 +/- 5 nm). Comme pour la progestérone, le busulfan n'est pas retrouvé dans la phase aqueuse à une concentration supérieure à sa solubilité. En effet, aucun cristal n'est visible par microscopie optique dans la phase aqueuse après encapsulation. Or, le busulfan étant quasiment insoluble dans l'eau, cela indique une incorporation du busulfan dans les nanocapsules.

### B) Suspension de busulfan (à une concentration de 0,50 mg/ml)

Une suspension de particules à 0,50 mg/l est préparée dans les mêmes conditions que précédemment après avoir solubilisé 50 mg de busulfan dans 1 ml de N,N-diméthyl-acétamide. On prélève 175 mg de cette solution que l'on ajoute aux 504 mg de Labrafac® . On pèse également 75 mg de Lipoïd® S75-3, 504 mg de Solutol® , 3,829 g d'eau et 88 mg de chlorure de sodium. L'émulsion initiale est donc à une concentration de 1,76 mg/ml d'émulsion. L'ensemble est réuni dans un même bêcher et placé sous agitation magnétique. Un chauffage est appliqué jusqu'à atteindre une température de 85°C. Toujours sous agitation magnétique, on laisse refroidir le système jusqu'à une température de 60°C. Ce cycle (entre 85°C et 60°C) est réalisé trois fois. Au dernier refroidissement, on effectue une trempe à 70°C en jetant 12,5 ml d'eau distillée à 2°C +/- 1°C. Le système est alors maintenu sous agitation magnétique pendant 5 min. La concentration finale, c'est-à-dire après trempage donc dilution, est de 0,50 mg / ml.

### Exemple 5 : Influence de la nature du corps gras sur la température d'inversion de phase

On compare le Labrafac® , huile composée de triglycérides des acides capriques et capryliques, avec des esters d'acides gras. On a pu mettre en évidence l'importance de la taille de leurs groupements terminaux sur la température d'inversion de phase. On observe une augmentation de la température d'inversion de phase avec une augmentation de la taille des groupements. Ainsi, dans la série des myristates, le changement d'aspect est visible à 69,5°C pour l'ester éthylique, à 71,5°C pour l'ester isopropylique et à 86,5°C pour l'ester octyldodécylique. Cette augmentation signifie que nous obtenons plus facilement une émulsion huile dans eau lorsque l'huile possède un indice HLB plus faible (plus lipophile). En effet, ce caractère lipophile plus marqué entraîne une accentuation des liaisons hydrophobes entre l'agent tensio-actif et l'huile, et il faut donc plus d'énergie pour inverser ce système. Par ailleurs, la longueur de la chaîne carbonée de l'acide gras n'influence pas la taille des particules, ni la température d'inversion de phase (entre C₁₄ et C₁₈). Il semble cependant que la double liaison présente dans l'oléate d'éthyle augmente sensiblement la température d'inversion de phase.

Les résultats sont consignés dans le tableau ci-dessous.

**TABLEAU IV**

| **Huiles** | **Nb de carbone (AcGras)** | **Doubles liaisons** | **T° changement d'aspect (°C)** | **Taille des particules (nm)** |
|---|---|---|---|---|
| Labrafac® lipophile | 8/10 | 0 | 77,0 | 43 ± 7 |
| Palmitate d'éthyle | 16 | 0 | 69,0 | 37 ± 15 |
| Oléate d'éthyle | 18 | 1 | 71,5 | 41 ± 5 |
| Myristate d'éthyle | 14 | 0 | 69,5 | 35 ± 13 |
| Myristate d'isopropyle | 14 | 0 | 71,5 | 44 ± 23 |
| Myristate d'octyl dodécyle | 14 | 0 | 86,5 | 42 ± 16 |

La valeur du HLB du corps gras ne semble pas affecter la taille des particules de manière significative.

### Exemple 6: Influence de la nature du tensio-actif lipophile sur la taille des nanocapsules

Différents types de lécithine dont les proportions en phosphatidylcholine varient de 40 à 90 % ont été utilisés. La taille moyenne des particules augmente avec la teneur en phosphatidylcholine dans la lécithine (tableau V ci-dessous). En effet, pour 40 % de phosphatidylcholine, la taille des nanocapsules est de 35 +/- 8 nm. tandis qu'elle est respectivement de 43 +/- 7 nm et 78 +/- 12 nm pour une proportion de 75 et 90 % de phosphatidylcholine dans la lécithine. En revanche, l'utilisation de molécules chargées n'a pas permis d'obtenir des nanocapsules.

**TABLEAU V**

| **Type de Lipoïd** | **% de phosphatidylcholine** | **Taille moyenne des particules (nm)** |
|---|---|---|
| Lipoïd® S45 | 40 | 35 ± 8 |
| Lipoïd® S75-3 | 69 | 43 ± 7 |
| Lipoïd® S100 | 90 | 78 ± 12 |
| Lipoïd® EPC | 98 | 61 ± 12 |
| Lipoïd® E80 | 80 | 72 ± 18 |

### Exemple 7 : Nanocapsules lipidiques présentant un principe actif hydrosoluble fixé à leur surface.

On prépare 500 mg d'une dispersion de nanocapsules lipidiques non chargées en principe actif, telles que décrites dans l'exemple 1, en utilisant la formulation suivante :
- Lipoïd® S 75-3 : 1,51 % massique
- Labrafac® W 1.1349 : 10,08 % massique
- Solutol® HS15 : 10,08 % massique
- Eau : 76,6 % massique
- NaCl : 1,76 % massique

Les nanocapsules lipidiques obtenues présentent une taille de 43 ± 7 nm. 50 mg de la dispersion de nanocapsules lipidiques obtenues sont dilués dans 1 ml d'eau et incubés sous agitation douce avec une solution aqueuse contenant 50 µg d'ADN (pSV β-galactosidase, Promega, France) pendant une heure en présence d'un mélange d'histones issues de thymus de veau (Boehringer Mannheim, Allemagne). On obtient des nanocapsules lipidiques présentant des molécules d'ADN condensées avec les protéines, adsorbées à leur surface.

## Revendications

1. Nanocapsules de taille moyenne inférieure à 150 nm, de préférence inférieure à 100 nm, de préférence encore inférieure à 50 nm, constituées d'un coeur essentiellement lipidique liquide ou semi-liquide à température ambiante, enrobé d'un film essentiellement lipidique solide à température ambiante.

2. Nanocapsules lipidiques selon la revendication 1, **caractérisées en ce que** leur indice de polydispersité est compris entre 5 et 15 %.

3. Nanocapsules lipidiques selon la revendication 1 ou 2, **caractérisées en ce que** l'épaisseur du film solide est comprise entre 2 et 10 nm.

4. Nanocapsules lipidiques selon l'une des revendications 1 à 3, **caractérisées en ce que** le coeur des nanocapsules est essentiellement constitué d'un corps gras, tel qu'un triglycéride ou un ester d'acide gras, représentant 20 à 60 %, de préférence 25 à 50 % en poids des nanocapsules.

5. Nanocapsules lipidiques selon la revendication 4, **caractérisées en ce que** le triglycéride constituant le coeur des nanocapsules est choisi parmi les triglycérides en C₈ à C₁₂, par exemple des triglycérides des acides capriques et capryliques et leurs mélanges.

6. Nanocapsules lipidiques selon la revendication 4, **caractérisées en ce que** l'ester d'acide gras constituant le coeur des nanocapsules est choisi parmi les esters d'acide gras en C₈ à C₁₈, par exemple le palmitate d'éthyle, l'oléate d'éthyle, le myristate d'éthyle, le myristate d'isopropyle, le myristate d'octyldodécyle, et leurs mélanges.

7. Nanocapsules lipidiques selon la revendication 6, **caractérisées en ce que** l'ester d'acide gras est en C₈ à C₁₂.

8. Nanocapsules lipidiques selon l'une des revendications 1 à 7, **caractérisées en ce que** le film solide est essentiellement constitué d'un tensio-actif lipophile.

9. Nanocapsules selon la revendication 8, **caractérisées en ce que** le rapport corps gras / composé tensio-actif lipophile est choisi entre 1 et 15, de préférence entre 1,5 et 13, plus préférentiellement entre 3 et 8.

10. Nanocapsules lipidiques selon la revendication 8 ou 9, **caractérisées en ce que** le tensio-actif lipophile est une lécithine dont la proportion en phosphatidylcholine est comprise entre 40 et 90 %.

11. Nanocapsules lipidiques selon l'une des revendications 1 à 10, **caractérisées en ce que** le film solide contient en outre un tensio-actif hydrophile non ionique par exemple le Solutol® HS 15 représentant 2 à 10 % en poids des nanocapsules.

12. Nanocapsules lipidiques selon l'une des revendications 1 à 11, **caractérisées en ce que** qu'elles contiennent un principe pharmaceutiquement actif.

13. Procédé de préparation des nanocapsules selon l'une quelconque des revendications 1 à 12, qui implique les opérations consistant à :
a)
- préparer une émulsion huile/eau contenant une phase grasse huileuse, un tensio-actif hydrophile non ionique, un tensio-actif lipophile solide à 20°C, et éventuellement un principe pharmaceutiquement actif soluble ou dispersible en phase grasse huileuse, ou un principe pharmaceutiquement actif soluble ou dispersible en phase aqueuse.
- provoquer l'inversion de phase de ladite émulsion huile/eau par augmentation de la température jusqu'à une température T₂ supérieure à la température d'inversion de phase (TIP) pour obtenir une émulsion eau/huile, suivie d'une diminution de la température jusqu'à une température T₁, T₁ < TIP < T₂.
- effectuer au moins un ou plusieurs cycles de température autour de la zone d'inversion de phase entre T₁ et T₂, jusqu'à observer une suspension translucide,
b) effectuer la trempe de l'émulsion huile/eau à une température voisine de T₁, de préférence supérieure à T₁, pour obtenir des nanocapsules stables.

14. Procédé selon la revendication 13, **caractérisé en ce que** la phase grasse huileuse est un triglycéride en C₈ à C₁₂, par exemple des triglycérides des acides capriques et capryliques et leurs mélanges ou un ester d'acide gras en C₈ à C₁₈, par exemple le palmitate d'éthyle, l'oléate d'éthyle, le myristate d'éthyle, le myristate d'isopropyle, le myristate d'octyldodécyle, et leurs mélanges.

15. Procédé selon la revendication 13 ou 14, **caractérisé en ce que** le tensio-actif hydrophile non ionique est le Solutol® HS 15.

16. Procédé selon l'une des revendications 13 à 15, **caractérisé en ce que** le tensio-actif lipophile est une lécithine dont la proportion en phosphatidylcholine est comprise entre 40 et 90 %, par exemple le Labrafac® WL 1349.

17. Procédé selon l'une des revendications 13 à 16, **caractérisé en ce que** l'émulsion huile/eau contient :
1 à 3 % de tensio-actif lipophile,
5 à 15 % de tensio-actif hydrophile,
5 à 15 % de corps gras huileux,
64 à 89 % d'eau,
les pourcentages étant exprimés en poids.

18. Procédé selon l'une des revendications 13 à 17, **caractérisé en ce que** l'émulsion huile/eau contient en outre de 1 à 4 % d'un sel, tel que le chlorure de sodium.

19. Procédé selon l'une des revendications 13 à 18, **caractérisé en ce que** le tensio-actif lipophile est solide à 37°C.

20. Procédé de préparation de nanocapsules selon l'une des revendications 13 à 19, **caractérisé en ce qu'**un principe pharmaceutiquement actif hydrosoluble est adsorbé à la surface libre des nanocapsules stables obtenues à l'issue de l'étape b).

21. Utilisation des nanocapsules selon l'une des revendications 1 à 12, pour la fabrication d'un médicament administré par voie injectable, notamment intraveineuse, par voie orale ou par voie nasale.

## Patentansprüche

1. Nanokapseln mit einer mittleren Größe von unter 150 nm, vorzugsweise unter 100 nm, noch mehr bevorzugt unter 50 nm, welche aus einem im wesentlichen lipidischen, bei Umgebungstemperatur flüssigen oder halbflüssigen Kern; welcher von einem im wesentlichen lipidischen, bei Umgebungstemperatur festen Film umhüllt ist, gebildet werden.

2. Lipidische Nanokapseln nach Anspruch 1, **dadurch gekennzeichnet, dass** ihr Polydispersitätsindex zwischen 5 und 15% beträgt.

3. Lipidische Nanokapseln nach Anspruch 1 oder 2, **dadurch gekennzeichnet, dass** die Dicke des festen Films zwischen 2 und 10 nm beträgt.

4. Lipidische Nanokapseln nach einem der Ansprüche 1 bis 3, **dadurch gekennzeichnet, dass** der Kern der Nanokapseln im wesentlichen aus einem Fett, wie einem Triglycerid oder einem Fettsäureester, welches bzw. welcher 20 bis 60 Gew.-%, vorzugsweise 25 bis 50 Gew.-% der Nanokapseln ausmacht, gebildet wird.

5. Lipidische Nanokapseln nach Anspruch 4, **dadurch gekennzeichnet, dass** das Triglycerid, welches den Kern der Nanokapseln bildet, ausgewählt wird unter den C.₈- bis C₁₂-Triglyceriden, beispielsweise Triglyceriden der Caprin- und Caprylsäure und deren Mischungen.

6. Lipidische Nanokapseln nach Anspruch 4, **dadurch gekennzeichnet**, der Fettsäureester, welcher den Kern der Nanokapseln bildet, ausgewählt wird unter den C₈- bis C₁₈-Fettsäureestern, beispielsweise Ethylpalmitat, Ethyloleat, Ethylmyristat, Isopropylmyristat, Octyldodecylmyristat und deren Mischungen.

7. Lipidische Nanokapseln nach Anspruch 6, **dadurch gekennzeichnet, dass** der Fettsäureester ein C₈- bis C₁₂-Fettsäureester ist.

8. Lipidische Nanokapseln nach einem der Ansprüche 1 bis 7, **dadurch gekennzeichnet, dass** der feste Film im wesentlichen aus einer lipophilen grenzflächenaktiven Substanz gebildet wird.

9. Nanokapseln nach Anspruch 8, **dadurch gekennzeichnet, dass** das Verhältnis Fett/lipophile grenzflächenaktive Verbindung zwischen 1 und 15, vorzugsweise zwischen 1,5 und 13, mehr bevorzugt zwischen 3 und 8 ausgewählt wird.

10. Lipidische Nanokapseln nach Anspruch 8 oder 9, **dadurch gekennzeichnet, dass** die lipophile grenzflächenaktive Substanz ein Lecithin ist, dessen Anteil an Phosphatidylcholin zwischen 40 und 90% beträgt.

11. Lipidische Nanokapseln nach einem der Ansprüche 1 bis 10, **dadurch gekennzeichnet, dass** der feste Film außerdem eine hydrophile, nicht-ionische grenzflächenaktive Substanz, beispielsweise Solutol® HS 15, welche 2 bis 10 Gew.-% der Nanokapseln ausmacht, enthält.

12. Lipidische Nanokapseln nach einem der Ansprüche 1 bis 11, **dadurch gekennzeichnet, dass** diese einen pharmazeutisch wirksamen Wirkstoff enthalten.

13. Verfahren zur Herstellung der Nanokapseln nach einem der Ansprüche 1 bis 12, welches die Verfahrensschritte umfasst, welche darin bestehen:
a)
- eine Öl/Wasser-Emulsion herzustellen, die eine ölartige Fettphase, eine hydrophile, nicht-ionische grenzflächenaktive Substanz, eine bei 20°C feste lipophile grenzflächenaktive Substanz und gegebenenfalls einen pharmazeutisch wirksamen Wirkstoff, welcher in einer ölartigen Fettphase löslich oder dispergierbar ist, oder einen pharmazeutisch wirksamen Wirkstoff, welcher in einer wässrigen Phase löslich oder dispergierbar ist, enthält,
- die Phasenumkehr der Öl/Wasser-Emulsion durch Erhöhung der Temperatur bis auf eine Temperatur T₂, welche höher als die Phasenumkehrtemperatur (TIP) ist, hervorzurufen, um eine Wasser/Öl-Emulsion zu erhalten, gefolgt von einer Verringerung der Temperatur bis auf eine Temperatur T₁, wobei T₁ < TIP < T₂,
- wenigstens einen oder mehrere Temperaturzyklen um die Phasenumkehrzone zwischen T₁ und T₂ auszuführen, bis eine durchscheinende Suspension beobachtet wird,
b) die Abschreckung der Öl/Wasser-Emulsion auf eine Temperatur nahe T₁, vorzugsweise über T₁ auszuführen, um stabile Nanokapseln zu erhalten,

14. Verfahren nach Anspruch 13, **dadurch gekennzeichnet, dass** die ölartige Fettphase ein C₈- bis C₁₂-Triglycerid ist, beispielsweise Triglyceride der Caprin- und Caprylsäure und deren Mischungen oder ein C₈-C₁₈-Fettsäureester, beispielsweise Ethylpalmitat, Ethyloleat, Ethylmyristat, Isopropylmyristat, Octyldodecylmyristat und deren Mischungen.

15. Verfahren nach Anspruch 13 oder 14, **dadurch gekennzeichnet, dass** die hydrophile, nicht-ionische grenzflächenaktive Substanz Solutol® HS 15 ist.

16. Verfahren nach einem der Ansprüche 13 bis 15, **dadurch gekennzeichnet, dass** die lipophile grenzflächenaktive Substanz ein Lecithin, dessen Anteil an Phosphatidylcholin zwischen 40 und 90% beträgt, beispielsweise Labrafac® WL 1349, ist.

17. Verfahren nach einem der Ansprüche 13 bis 16, **dadurch gekennzeichnet, dass** die Öl/Wasser-Emulsion enthält:
1 bis 3% lipophile grenzflächenaktive Substanz,
5 bis 15% hydrophile grenzflächenaktive Substanz,
5 bis 15% ölartiges Fett,
64 bis 89% Wasser,
wobei die Prozentsätze auf das Gewicht bezogen ausgedrückt sind.

18. Verfahren nach einem der Ansprüche 13 bis 17, **dadurch gekennzeichnet, dass** die Öl/Wasser-Emulsion außerdem 1 bis 4% eines Salzes, wie Natriumchlorid, enthält.

19. Verfahren nach einem der Ansprüche 13 bis 18, **dadurch gekennzeichnet, dass** die lipophile grenzflächenaktive Substanz bei 37°C fest ist.

20. Verfahren zur Herstellung von Nanokapseln nach einem der Ansprüche 13 bis 19, **dadurch gekennzeichnet, dass** ein wasserlöslicher, pharmazeutisch wirksamer Wirkstoff auf der freien Oberfläche der stabilen Nanokapseln, die am Ende von Schritt b) erhalten werden, adsorbiert wird.

21. Verwendung der Nanokapseln nach einem der Ansprüche 1 bis 12 für die Herstellung eines Arzneimittels, welches durch Injektion, insbesondere auf intravenösem Wege, auf oralem Wege oder auf nasalem Wege verabreicht wird.

## Claims

1. Nanocapsules with an average size of less than 150 nm, preferably less than 100 nm and more preferably less than 50 nm, consisting of an essentially lipid core that is liquid or semiliquid at room temperature, coated with an essentially lipid film that is solid at room temperature.

2. Lipid nanocapsules according to Claim 1, **characterized in that** their polydispersity index is between 5% and 15%.

3. Lipid nanocapsules according to Claim 1 or 2, **characterized in that** the thickness of the solid film is between 2 and 10 nm.

4. Lipid nanocapsules according to one of Claims 1 to 3, **characterized in that** the core of the nanocapsules consists essentially of a fatty substance, such as a triglyceride or a fatty acid ester, representing 20% to 60% and preferably 25% to 50% by weight of the nanocapsules.

5. Lipid nanocapsules according to Claim 4, **characterized in that** the triglyceride constituting the core of the nanocapsules is chosen from C₈ to C₁₂ triglycerides, for example capric and caprylic acid triglycerides, and mixtures thereof.

6. Lipid nanocapsules according to Claim 4, **characterized in that** the fatty acid ester constituting the core of the nanocapsules is chosen from C₈ to C₁₈ fatty acid esters, for example ethyl palmitate, ethyl oleate, ethyl myristate, isopropyl myristate and octyldodecyl myristate, and mixtures thereof.

7. Lipid nanocapsules according to Claim 6, **characterized in that** the fatty acid ester is C₈ to C₁₂.

8. Lipid nanocapsules according to one of Claims 1 to 7, **characterized in that** the solid film consists essentially of a lipophilic surfactant.

9. Nanocapsules according to Claim 8, **characterized in that** the fatty substance/lipophilic surfactant compound ratio is chosen between 1 and 15, preferably between 1.5 and 13 and more preferably between 3 and 8.

10. Lipid nanocapsules according to Claim 8 or 9, **characterized in that** the lipophilic surfactant is a lecithin whose phosphatidylcholine proportion is between 40% and 90%.

11. Lipid nanocapsules according to one of Claims 1 to 10, **characterized in that** the solid film also contains a nonionic hydrophilic surfactant, for example Solutol® HS 15, representing 2% to 10% by weight of the nanocapsules.

12. Lipid nanocapsules according to one of Claims 1 to 11, **characterized in that** they contain a pharmaceutically active principle.

13. Process for preparing the nanocapsules according to any one of Claims 1 to 12, which involves the operations consisting in:
a)
- preparing an oil/water emulsion containing an oily fatty phase, a nonionic hydrophilic surfactant, a lipophilic surfactant that is solid at 20°C and optionally a pharmaceutically active principle that is soluble or dispersible in the oily fatty phase, or a pharmaceutically active principle that is soluble or dispersible in the aqueous phase,
- bringing about the phase inversion of said oil/water emulsion by increasing the temperature up to a temperature T₂ above the phase inversion temperature (PIT) to obtain a water/oil emulsion, followed by a reduction in the temperature down to a temperature T₁, T₁ < PIT < T₂,
- carrying out at least one or more temperature cycles around the.phase inversion zone between T₁ and T₂, until a translucent suspension is observed,
b) annealing the oil/water emulsion at a temperature in the region of T₁, preferably greater than T₁, to obtain stable nanocapsules.

14. Process according to Claim 13, **characterized in that** the oily fatty phase is a C₈ to C₁₂ triglyceride, for example capric and caprylic acid triglycerides and mixtures thereof or a C₈ to C₁₈ fatty acid ester, for example ethyl palmitate, ethyl oleate, ethyl myristate, isopropyl myristate or octyldodecyl myristate, and mixtures thereof.

15. Process according to Claim 13 or 14, **characterized in that** the nonionic hydrophilic surfactant is Solutol® HS 15.

16. Process according to one of Claims 13 to 15, **characterized in that** the lipophilic surfactant is a lecithin whose phosphatidylcholine proportion is between 40% and 90%, for example Labrafac® WL 1349.

17. Process according to one of Claims 13 to 16, **characterized in that** the oil/water emulsion contains:
1% to 3% of lipophilic surfactant,
5% to 15% of hydrophilic surfactant,
5% to 15% of oily fatty substance,
64% to 89% of water,
the percentages being expressed on a weight basis.

18. Process according to one of Claims 13 to 17, **characterized in that** the oil/water emulsion also contains from 1% to 4% of a salt, such as sodium chloride.

19. Process according to one of Claims 13 to 18, **characterized in that** the lipophilic surfactant is solid at 37°C.

20. Process for preparing nanocapsules according to one of Claims 13 to 19, **characterized in that** a water-soluble pharmaceutically active principle is adsorbed onto the free surface of the stable nanocapsules obtained after step b).

21. Use of the nanocapsules according to one of Claims 1 to 12, for the manufacture of a medicament administered by injection, especially by intravenous injection, orally or nasally.
